# EUROPEAN PATENT SPECIFICATION

(11) **EP 4 418 865 B1**
(45) Date of publication and mention of the grant of the patent: **10.12.2025**
(21) Application number: 22798167.7
(22) Date of filing: 14.10.2022
(51) Int. Cl.: A23D 7/005, A23L 2/52, A23L 33/15, A23L 33/155

(54) **LIQUID COMPOSITION CONTAINING A COMBINATION OF VITAMINS A, D, E AND K IN A LIQUID MATRIX**
FLÜSSIGE ZUSAMMENSETZUNG MIT EINER KOMBINATION DER VITAMINE A, D, E UND K IN EINER FLÜSSIGEN MATRIX
COMPOSITION LIQUIDE CONTENANT UNE COMBINAISON DE VITAMINES A, D, E ET K DANS UNE MATRICE LIQUIDE

(30) Priority: 19.10.2021 EP 21203508
(43) Date of publication of application: 28.08.2024
(73) Proprietor: Lipoid GmbH, 67065 Ludwigshafen (DE); IPSICO GmbH, 68199 Mannheim (DE)
(72) Inventor: HEIDECKE, Christoph, 67065 Ludwigshafen (DE); LORENZ, Luise, 67065 Ludwigshafen (DE); REBMANN, Balder, 67065 Ludwigshafen (DE); BREITENBACH, Jörg, 68199 Mannheim (DE)
(74) Representative: Meissner Bolte Partnerschaft mbB
(86) International application number: PCT/EP2022/078707
(87) International publication number: WO 2023/066815

(56) References cited:
- EP-A1- 2 522 350
- CN-A- 103 040 845
- US-A- 6 048 886
- US-A1- 2010 183 770
- US-B1- 10 722 465

## Description

The present invention relates to a liquid composition containing a combination of vitamins A, D, E and K or a suitable provitamin thereof in a liquid matrix, where the total amount of said vitamins and provitamins is at least 0.3% by weight of the total compositions. The invention further relates to the use of this vitamin formulation in the treatment of an existing or impending vitamin deficiency condition concerning at least one of vitamin A, D, E or K, in particular due to vitamin maldigestion.

The present invention further relates to the use of the compositions for dietary purposes, including e.g. food supplementation and food for special purposes, such as a balanced diet. The present invention further relates to the use of the compositions for medical purposes and for cosmetic purposes.

Fat-soluble vitamins, like vitamins A, D, E and K or their provitamins, are emulsified, pinocytotically absorbed into the mucosal cells and slowly absorbed into the lymph with the chylomicrons over a period of hours. These transport processes are the basis for enterohepatic circulation. Disturbances in these transport processes and thus in absorption are referred to as malabsorption.

Vitamin maldigestion in mammals, in particular in human beings, is a symptom of certain diseases such as cystic fibrosis. Cystic fibrosis is an autosomal recessive inherited metabolic disease, the cause of which is a mutational malfunction of chloride channels of certain cells of the body, resulting in a change in the secretory composition of all exocrine glands of the patient. The patient's affected cells are unable to draw water into the surrounding tissues by osmosis, resulting in low water content of the bronchial secretions as well as the secretions of the pancreas, liver (bile), internal sex organs and accessory sex glands, and small intestine. As a result, the secretions become viscous and various types of dysfunction may occur in the affected organs. Cystic fibrosis is therefore a multisystem disease. Typical symptoms of cystic fibrosis in the digestive system are maldigestion, malnutrition, indigestion, chronic diarrhea, and underweight. This also includes a reduced absorption capacity for especially fat-soluble vitamins and provitamins, which can lead to chronic deficiencies. In the treatment of cystic fibrosis patients, attempts are made to compensate for this by administering larger amounts of these (pro-)vitamins to the patient.

Vitamin maldigestion in mammals may also be caused by digestive disorders associated with a deficiency or loss of bile acid or digestive enzymes of the pancreas and/or the function of the small intestine. Digestive disorders occur in numerous diseases of the gastrointestinal tract, liver and pancreas, such as gastric or intestinal resection, pancreatitis, cystic fibrosis and disorders of intestinal flora, which may be caused by treatment with cytostatica in cancer treatment.

High-dose vitamin formulations are desirable to reduce the number of tablets or capsules to be taken by the patient. However, high concentrations of fat-soluble vitamins often lead to an unpleasant mouthfeel or bad taste. For example, high concentrations of β-carotene, the main representative of the A provitamins, often result in a taste that is described as "bland," "numb," or "old." While vitamin D preparations are often perceived as tasting bitter or metallic. Furthermore, it is known that formulations of fat-soluble vitamins, especially those containing vitamin D or (pro-)vitamin A, usually have a bad taste that can easily lead to vomiting, especially in the case of oil-based formulations. This poses a problem, especially for children, for the acceptance of formulations containing a high concentration of such vitamins and/or provitamins.

EP-A-1338271 describes a multivitamin solubilisate containing ascorbic acid and a fat-soluble vitamin (α-tocopherol, retinol or β-carotene). The multivitamin solubilisate is prepared by a three-step process comprising (a) the preparation of a first solubilisate by dissolving ascorbic acid in water and homogenizing the solution with a vegetable oil and polysorbate 80, (b) providing a second solubilisate by dissolving the fat-soluble vitamin in polysorbate 20 and mixing the first and the second solubilisate. The thus obtained multivitamin solubilisates contain at least 70% by weight of polysorbate emulsifiers.

A chewable composition containing one or more of vitamins A, D, E and K is described in DE 202004010021. This chewing mass is prepared by first mixing the vitamins with a high amount of emulsifier, such as polysorbate 80 or polysorbate 20, and stirring until a clear, homogeneous solubilisate is formed. The solubilisate is then included in a suitable thickening agent.

The use of emulsifiers, such as polysorbates, in the preparation of oral formulations generally has the physiological disadvantage that such compounds can negatively affect the digestive system and cause diarrhea, especially with repeated use. Such side effects are particularly detrimental after surgical procedures on the gastrointestinal tract or in patients in whom the digestive system is significantly impaired. Also, the administration of oil-containing preparations of solubilized fat-soluble vitamins, such as those commonly found in the form of oil-filled soft gelatin capsules, may be very detrimental to patients with an impaired digestive system and should be avoided.

EP 3292859 describes chewable or suckable compositions containing vitamins A, D, E and K or a provitamin thereof in non-solubilized form in a hydrocolloid gel which may contain a sweetener. The compositions are prepared by (a) forming a warm gel of water, the hydrocolloid, such as gelatin, and optionally the sweetener, (b) incorporating the vitamins and further ingredients into the hydrocolloid gel with stirring until a translucent gel is obtained and (c) forming the translucent gel to the desired dosage form. While the compositions described therein are stable, do not have an unpleasant taste and are even accepted by children, the compositions do not allow for individual dosage but are limited to defined single dosage forms. Moreover, the total concentration of the vitamins A, D, E and K in the formulation is comparatively low.

Orthomol^{®} Immune is a commercial multivitamin nutritional product for special medical purpose, i.e. for balanced diet, which contains vitamins A, mainly from β-carotene, in combinations with vitamins D, E and K and other vitamins. The dosage form of Orthomol^{®} Immune is a combination of a drinking bottle and a tablet. Individual dosages are not possible.

WO 2004/047791 describes emulsion type liquid concentrate compositions of a fatty component containing a combination of a phospholipid and a polyol or carbohydrate in a specific ratio. The liquid concentrate can be easily diluted with water. WO 2004/047791 does not describe liquid co-formulations of all vitamins A, D, E and K.

US 10,722,465 describes an aqueous, intra-oral, transparent nanoemulsion blend that contains both oil- and water-soluble components of a vitamin supplement, where the nanoemulsion contains at least two different bilayer water-core liposome components and at least one monolayer surfactant bound particle component. The composition may contain small amounts of vitamins A, D, E and K. The formulations are difficult to prepare and require conventional emulsifiers, such as polysorbates and tocopherol polyethylenglycol succinat, also referred to as Vitamin E TPGS. Due to the considerable amount of water contained therein they are likely not autosterilic and require sterilization. Moreover, the formulations contain considerable amounts of ethanol, which may be problematic for health reasons.

US 2010/0183770 describe oil-in-water emulsions containing water, fat-soluble ingredients, such as fat-soluble vitamins and emulsifiers selected from the group of caseinates. In addition, they contain a considerable amount of an oil-phase, which renders them unsuitable for oral administration as such. The oil-in-water emulsions are suggested for supplementing dairy products, such as milk or milk products with vitamins. Caseinates are potent allergens and are the most common triggers of cow's milk allergies. Moreover, these oil-in-water emulsions are likely not autosterilic and require sterilization.

CN 103040845 describe injectable aqueous fat emulsions for supplementation of fat-soluble vitamins, such as vitamins A, D, E and K. The total concentration of these fat-soluble vitamins is at most 14 mg/10 ml. The formulations are not particularly suitable for oral administration. Moreover, these oil-in-water emulsions are likely not autosterilic and require sterilization.

So far, compositions containing a combination of vitamins A, D, E and K in a total amount of at least 0.3% by weight, based on the total weight of the composition, in a single liquid composition are difficult prepare and no formulations containing a combination of vitamins A, D, E and K have been described, which are autosterilic. Indeed, such a combination is difficult to co-formulate in a single liquid composition that is stable and can be easily diluted with water. Rather, specific formulation approaches are required to obtain stable formulations, which in turn, however, are associated with problems such as a high production effort, limited application and/or incompatibility when administered orally as such. Moreover, stable formulations containing these vitamins in higher amounts than 0.3% by weight will require conventional emulsifiers, which may be problematic for the reasons outlined above.

It is, therefore, desirable to provide a high-dose vitamin composition containing vitamin A, vitamin D, vitamin E and vitamin K and optionally a carotenoid, such as β-carotene, in the form of a liquid vitamin composition, which is stable at prolonged storage and which can be administered orally as such or as dilution with water, and which can be incorporated into formulations for oral or topical administration. Moreover, the composition should be gentle on the patients' digestive system and have a good taste despite being high in fat-soluble vitamins, such as vitamin A, vitamin D, vitamin E and vitamin K and optionally a carotenoid, such as β-carotene. Moreover, the compositions should be autosterilic and do not require sterilization. Last but not least, the compositions should be easy to produce, especially without the need for artificial emulsifiers or the production of liposomes.

It was surprisingly found that these objectives are met by the liquid compositions, which contain the following components a) to h) and optionally contain further ingredients, such as carotenoids, e.g. ß-carotene, and/or zinc salts.

The liquid compositions of the invention contain the following components a) to h):
a) at least one of vitamin A and esters of vitamin A,
b) at least one of vitamin D and provitamin D,
c) at least one of vitamin E and esters of vitamin E,
d) at least one of vitamin K and provitamin K,
e) 40 to 95% by weight, in particular 45 to 90% by weight, especially 50 to 85% by weight, based on the total weight of the liquid composition, of at least one water miscible polyol,
f) 1 to 40% by weight, in particular 2 to 40% by weight, especially 5 to 35% by weight, based on the total weight of the liquid composition, of at least one vegetable triglyceride;
g) 3 to 30% by weight, in particular 6 to 25% by weight, especially 7 to 20% by weight, based on the total weight of the liquid composition, water; and
h) 0.5 to 10% by weight, in particular 0.8 to 8% by weight, especially 1 to 7% by weight, based on the total weight of the liquid composition, of a phospholipid composition containing phosphatidylcholine;
i) optionally one or more carotenoids or esters thereof, e.g. ß-carotene;
wherein the total amount of components a) to d) is at least 0.3% by weight, in particular at least 0.4% by weight, especially at least 0.5% by weight, based on the total weight of the liquid composition, e.g. in an amount in the range of 0.3 to 20% by weight, in particular in the range of 0.3 to 15% or in the range of 0.4 to 15% by weight, especially in the range of 0.4 to 10% by weight or the range of 0.5 to 10% by weight, based on the total weight of the liquid composition.

The compositions of the present invention are stable upon storage and do not form inhomogeneity, such as phase separation, creaming, sediments or other inhomogeneity even after prolonged storage. The compositions can be easily diluted with water, whereby translucent oil-in-water emulsions are formed with very small oil droplets dispersed in the continuous aqueous phase. The compositions of the present invention are autosterilic and do not require sterilization. The compositions of the invention have an acceptable taste, as the combination of components e) to h) will mask the bad taste of components a) to d) and the optionally present carotenoid i), such as ß-carotene. Moreover, the compositions of the invention provide for improved bioavailability of the vitamins A, D, E and K, because the components a) to d) and the optionally present carotenoid are present in solubilized form. Therefore, the compositions of the present invention are particularly useful for oral administration at individual dosages. Moreover, the compositions of the present invention can be used as ingredients of formulations for oral administration, including in particular dosage forms for oral administration, e.g. capsules, such as soft-capsules and hard capsules. In addition, the compositions of the invention are compatible with common pharmaceutical or cosmetic excipients for topical formulations and, therefore, can be used in formulations for topical applications or administration, respectively. Since the compositions of the invention are stable liquids, they are also suitable for parenteral administration, either as such or as a dilution in a liquid excipient suitable for parenteral administration. It is believed that the particular advantages of the present invention are due to the combination and balanced relative amounts of components (e) to (h).

The liquid compositions according to the invention are characterized by a very high content of the fat-soluble vitamins A, D, E and K, or the respective provitamins, which, moreover, are predominantly present in a solubilized form and thus represent a significant surface area that will come into contact with the tongue as a taste organ during consumption. Nevertheless, unlike known vitamin formulations, the compositions surprisingly do not have an unpleasant taste. Furthermore, it was surprising that a significant amount of a zinc salt can be incorporated into the composition of the invention, without imparting the stability or the taste thereof. In particular, the typical unpleasant taste of zinc salts, which is described as "sharp metallic salty" is not observed. All this represents an enormous advantage with regard to the acceptance of the compositions of the invention by patients, and especially by children, or makes acceptance possible in the first place.

The liquid compositions of the invention contain a high amount of the fat-soluble vitamins A, D, E and K, or the respective provitamins. As a result, 100% or more of the recommended daily allowance of these vitamins can be administered with an amount acceptable to the patient (e.g. 20 g of the mass) (see e.g. EU-RDA ("Recommended Daily Allowance") according to Directive 2008/100/EC). The liquid compositions promote the absorption of the fat-soluble a), b), c) and d) and i), if present, into the mammalian organism and thus provide a good bioavailability of the vitamins. Due to the good bioavailability of the ingredients a) to d) and the optionally present carotenoid, the compositions of the present invention are particularly useful for the treatment of an existing or impending vitamin deficiency condition concerning at least one of vitamin A, D, E or K. Therefore, a further aspect relates to the use of the compositions as defined herein in the treatment of an existing or impending vitamin deficiency condition concerning at least one of vitamin A, D, E or K.

Further aspects of the present invention are:
- The use of the compositions as described herein in a medical treatment of a disease or condition of a mammal, in particular in a human being, where the disease or condition is associated with or caused by an existing or impending vitamin deficiency condition in a mammal;
- The use of the compositions as described herein in the co-treatment of a disease in a mammal, in particular in a human being, with a therapeutic agent different from vitamin A, D, E and K, where the disease or the treatment with the therapeutic agents causes or may cause vitamin deficiency condition concerning at least one of vitamin A, D, E or K;
- The use of the compositions as described herein for dietary purposes, such as food supplementation or in food for special medical purposes, such as food for a balanced diet of a mammal, in particular of a human being;
- The use of the compositions as described herein in cosmetic formulations;
- A method of treatment of a disease or condition of a mammal, in particular of a human being, which comprises administering a composition of the invention to a mammal in need thereof, where the disease or condition is associated with or caused by an existing or impending vitamin deficiency condition in a human;
- A method of co-treatment of a disease in a mammal, in particular in a human being, where the mammal is treated with a therapeutic agent different from vitamin A, D, E and K, where the disease or the treatment with the therapeutic agents causes or may cause vitamin deficiency condition concerning at least one of vitamin A, D, E or K, which comprises administering a composition of the invention to a mammal in need thereof, i.e. to the mammal, which has been treated with a therapeutic agent different from vitamin A, D, E and K.

Here and in the following, the term "use for medical purposes" includes both the use of the compositions of the invention as a component of a medicinal product and its use as a component of a pharmaceutical product.

Here and in the following, the term "use for dietary purposes" includes both the use of the compositions of the invention, either as such or as a component of a formulation, for food supplementation and use of the compositions of the invention, either as such or as a component of a formulation, as a food for special medical purposes, such as a balanced diet.

Although the compositions of the present invention have multiple phases due to the different polarities of the ingredients e) and g) on one hand and the components a) to d), f) and optional component i) on the other hand, they are translucent liquids and not turbid. In other words, the compositions are virtually homogeneous.

As mentioned above, the compositions of the invention will form a translucent oil-in-water emulsion when they are diluted with water. In particular, the compositions of the present invention are characterized in that a dilution of the liquid composition in deionized water at 25°C and at a concentration of 1% by weight forms droplets having a Z average particle diameter of at most 500 nm, in particular at most 350 nm, e.g. in the range of 50 to 500 nm, in particular in the range of 50 to 350 nm, as determined by dynamic light scattering.

The component a) is selected from vitamin A including retinol (vitamin A1), retinoic acid (vitamin A acid, e.g., all-trans-retinoic acid and 13-cis-retinoic acid) and retinals (vitamin A aldehyde e.g., all-trans-retinal and 11-cis-retinal) and retinol esters, i.e. esters of retinol with fatty acid, in particular C₂ to C₂₀ fatty acids, such as retinol acetate, retinol propionate and retinol palmitate. Preference is given to retinol esters as component a). The concentration of the component a) in the composition of the invention is preferably in the range of 0.001 to 0.4% by weight, in particular in the range of 0.002 to 0.3% by weight and especially in the range of 0.003 to 0.2% by weight, based on the total weight of the liquid composition.

The component b) is selected from vitamin D and provitamin D. The term "vitamin D" refers to several chemical compounds, such as vitamin D3 (cholecalciferol, calciol), vitamin D2 (calciferol, ergocalciferol) and vitamin D4 (22,23-dihydroergocalciferol, saturated vitamin D2). In particular, "provitamin D" refers to provitamin D2 (ergosterol) and provitamin D3 (7-dehydrocholesterol). In the composition according to the invention, the component b) is preferably vitamin D3. The concentration of the component b) in the composition of the invention is preferably in the range of 0.00005 to 0.3% by weight, in particular in the range of 0.00007 to 0.25% by weight and especially in the range of 0.00008 to 0.2% by weight, based on the total weight of the liquid composition.

The component c) is selected from vitamin E and esters of vitamin E. The term "vitamin E" refers to several chemical compounds, such as tocopherols, e.g. α-tocopherol, β-tocopherol, γ-tocopherol, δ-tocopherol, 5,7-dimethyltocol and 7-methyltocol, tocomonoenols, e.g. α-tocomonoenol, β-tocomonoenol, γ-tocomonoenol and δ-tocomonoenol, tocotrienols, e.g. α-tocotrienol, β-tocotrienol, γ-tocotrienol, and δ-tocotrienol, and marine tocopherols ("marine-derived tocopherol" = MDT), e.g. α-MDT, β-MDT, γ-MDT and δ-MDT. Likewise, suitable are vitamin E esters, such as tocopheryl acetates, e.g. D,L-α-tocopheryl acetate and D-α-tocopheryl acetate, and tocopheryl succinates, e.g. D,L-α-tocopheryl succinate and D-α-tocopheryl succinate. In the composition according to the invention, the preferred component E is D,L-α-tocopheryl acetate or D-α-tocopheryl acetate. The concentration of the component c) in the composition of the invention is preferably in the range of 0.1 to 19% by weight, in particular in the range of 0.2 to 14% by weight and especially in the range of 0.3 to 9% by weight, based on the total weight of the liquid composition.

The component d) is selected from vitamin K and provitamin K. The term "vitamin K" or "coagulation vitamin" refers to several chemical compounds derived from 2-methyl-1,4-naphtoquinone, such as vitamin K1 (phylloquinone), vitamin K2 (menaquinone), vitamin K3 (menadione) and vitamin K4 (menadiol). In particular, vitamin K adducts, such as hydrogen sulfite vitamin K3 adduct, may be referred to as "provitamin K". In the composition according to the invention, the component d) is preferably vitamin K1. The concentration of the component d) in the composition of the invention is preferably in the range of 0.001 to 0.4% by weight, in particular in the range of 0.002 to 0.3% by weight and especially in the range of 0.003 to 0.2% by weight, based on the total weight of the liquid composition.

In particular, the concentrations of components a) to d) in the composition of the present invention are as follows:
a) 0.001 to 0.4% by weight, in particular 0.002 to 0.3% by weight and especially 0.003 to 0.2% by weight, based on the total weight of the liquid composition, of component a),
b) 0.00005 to 0.3% by weight, in particular 0.00007 to 0.25% by weight and especially 0.00008 to 0.2% by weight, based on the total weight of the liquid composition, of component b),
c) 0.1 to 19% by weight, in particular 0.2 to 14% by weight and especially 0.3 to 9% by weight, based on the total weight of the liquid composition, of component c),
d) 0.001 to 0.4% by weight, in particular 0.002 to 0.3% by weight and especially 0.003 to 0.2% by weight, based on the total weight of the liquid composition, of component d);
where the total concentration of components a), b), c) and d) is in the range of 0.3 to 20% by weight, in particular in the range of 0.3 to 15% by weight or in the range of 0.4 to 15% by weight, especially in the range of 0.4 to 10% by weight or in the range of 0.5 to 10% by weight, based on the total weight of the liquid composition.

The compositions of the invention further contain at least one polyol as a component e). For the purpose of the invention, any polyol is suitable, which is miscible with water and acceptable for oral administration, nutrition or dietary purposes. Suitable polyols are glycerol and water-soluble carbohydrates, in particular monosaccharides and disaccharides, such as glucose, fructose or saccharose. Preferably, the component e) comprises glycerol as a main constituent of component e). In particular, the component e) is glycerol. In combination with the water of component g), the component e) forms a polar phase within the composition of the invention. The total amount of the components e) and g) is typically in the range of 43 to 98% by weight, in particular in the range of 51 to 96.5% by weight and especially 57 to 94% by weight, based on the total weight of the liquid composition.

The compositions of the invention further contain at least one edible triglyceride as component f). In this context, the term "edible oil" refers to an oil, which is acceptable for oral administration, nutrition and/or dietary purposes. Suitable oils are vegetable oils, in particular oils of seed-oil plants, such as sunflower oil, soybean oil, rapeseed oil, olive oil, coconut oil or safflower oil, grape seed oil or hemp oil, fractions of plant oils, such as medium chain triglycerides (MCT) and long chain triglycerides (LCT), but also oils of marine organisms, such as fish oil, krill oil or algae oil. Likewise, suitable are mixtures of the aforementioned oils, such as soybean oil/olive oil mixtures, soybean oil/safflower oil mixtures, soybean oil/MCT mixtures, inter-esterified mixtures of LCT and MCT, and soybean oil/MCT/olive oil/fish oil mixtures. The aforementioned oils mainly consist of triglycerides but may of course contain miner amounts of fatty acids. Typically, the oils consist of at least 99% by weight of triglycerides of fatty acids and optionally free fatty acids.

Preference is given to components f) which comprise a medium chain triglyceride as a main constituent.

As component f), preference is given to triglycerides having a melting point in the range of 0 to 40°C.

The amount of the component f) in the liquid composition of the present invention is preferably chosen such that the weight ratio of the component f) to the total weight of lipophilic components a), b) c) and d) and optionally i) is in the range of 1:1 to 100:1, in particular in the range of 2:1 to 50:1.

The composition of the invention further contains a phospholipid as a component h).

According to the invention, the phospholipid compositions contain a phosphatidylcholine, which mainly serves for the stabilization of the different phases in the composition of the invention, and which also assists in the stabilization of the oil droplets of the oil-in-water emulsions when the composition is diluted with water. Usually, phosphatidylcholine is the main component of the phospholipid compositions. Preferably, the content of phosphatidylcholine in the phospholipid composition is at least 45.0% by weight, in particular at least 50.0% by weight, especially at least 60.0% by weight or at least 70.0% by weight, based on the total weight of the phospholipid composition. Frequently, the content of phosphatidylcholine will not exceed 99.0% by weight, in particular 98.5% by weight, especially 98.0% by weight, based on the total weight of the phospholipid composition.

Depending on the amount of phosphatidylcholine, the phospholipid compositions may contain one or more further phospholipid compounds different from phosphatidylcholine, in particular one or more of the following phospholipid compounds: phosphatidylethanolamine, N-acylphosphatidylethanolamine and lysophosphatidylcholine. The total amount of phospholipid compounds different from phosphatidylcholine is typically in the range of 1.0 to 50.0% by weight, in particular in the range of 1.5 to 45.0% by weight, especially in the range of 2.0 to 40.0% by weight or in the range of 2.0 to 30.0% by weight, based on the total weight of the phospholipid composition. For example, the amount of lysophosphatidylcholine may be from traces up to 25.0% by weight, based on the total weight of the sunflower phospholipid composition. Likewise, the amount of phosphatidylethanolamine may be from traces up to 25.0% by weight, based on the total weight of the phospholipid composition and is frequently in the range of 1.0 to 20.0% by weight. Typically, the amount of N-acylphosphatidylethanolamine will not exceed 10.0% by weight and may be present from traces up to 10.0% by weight, based on the total weight of the phospholipid composition. In a particular group of embodiments, the phospholipid compositions contain an anionic phospholipid compound, such as phosphatidylglycerol, for example in an amount in the range of 0.1 to 2.0% by weight, based on the total weight of the phospholipid composition.

Furthermore, the phospholipid compositions may contain one or more fatty acid triglycerides and/or free fatty acids, which are residues from their production process. Typically, the total amount of triglycerides and free fatty acids will not exceed 10.0% by weight, based on the total weight of the phospholipid composition. Due to the presence of free fatty acids, the phospholipid compositions may have an acid number different from 0. Here and in the following, the acid number refers to the acid number in mg KOH/g. Usually, the acid number will not exceed a value of 50, in particular 40 and is frequently in the range of 0.01 to 35.

The phospholipid composition is typically obtained from vegetable or animal origin by extraction and purification processes. Suitable phospholipid compositions may be phospholipid compositions from egg, phospholipid compositions from sunflower and phospholipid compositions from soybean. Phospholipid composition obtained from vegetable origin may be obtained from genetically modified plants (GMO plants) or from plants obtained by conventional breeding (non-GMO) plants. Preferred phospholipid compositions are those of vegetable origin. Particular preference is given to phospholipid compositions from non-GMO sunflower and phospholipid compositions from non GMO soybean.

In the liquid compositions of the present invention, the weight ratio of the phosphatidylcholine in component h) to the component f) is preferably in the range of 1:20 to 1:1, in particular in the range of 1:15 to 1:1.5.

In addition to the aforementioned components a) to h), the liquid composition of the present invention may further contain one or more carotenoids and/or an ester thereof as a component i). Suitable carotenoids are ß-carotene (E 160a), astaxanthin (E 161), canthaxanthin (E 160g), capsanthin (E 160c), capsorubin (E 160c), lutein (E 161b), Lycopin (E 160d) and zeaxanthin (E 161h). Likewise, suitable are the esters of those carotenoids, which have OH groups, e.g. the esters of astaxanthin, Capsanthin, Capsorubin or Lutein, in particular the fatty acid esters thereof, such as the acetates, propionates, palmitates, stearates or oleates thereof. Particular preference is given to ß-carotene as component i). If present, the concentration of the carotenoid in the liquid composition is in the range of 0.01 to 10% by weight, in particular in the range of 0.02 to 5% by weight, based on the total weight of the liquid composition.

Due to the presence of the hydrophobic component f) and the hydrophilic components e) and g) the liquid compositions of the invention are capable of incorporating both highly polar substances and lipophilic substances. Highly polar substances suitable for the purpose of the invention include water-soluble, possibly solubilized vitamins, e.g. B vitamins, such as vitamin B6 and vitamin B12, and folic acid, amino acids e.g. L-tryptophan, cofactors, such as zinc compounds, vitamin C or a salt thereof, such as sodium ascorbate, and aminosulfonic acids, such as taurine. Further hydrophobic substances include phytosterols, naturally occurring polyphenols, flavonoids and flavones and ubiquinones.

In a particular group of embodiments, the liquid compositions of the present invention further contain a zinc salt, which is suitable for nutritional or dietary purposes or which is pharmaceutically acceptable. Examples of suitable zinc salt include zinc sulfate, zinc gluconate, zinc bisglycinate, zinc acetate, zinc hydroxide carbonate, zinc lactate, zinc ascorbate, in particular zinc L-ascorbate, zinc hydrogen ascorbate, zinc aspartate, zinc hydrogen aspartate, such as zinc DL-hydrogen aspartate or zinc L-hydrogen aspartate, zinc citrate, zinc oxide, zinc stearate, zinc orotate, zinc picolinate and the hydrates thereof. If present, the concentration of the zinc salt in the liquid composition is generally in the range of 0.01 to 0.2% by weight, based on the total weight of the liquid composition and calculated as elemental zinc.

In a further particular group of embodiments, the liquid compositions of the present invention further contain a water-soluble organic anti-oxidant. In this context, the term "water-soluble" means that the anti-oxidant has a solubility in water of at least 50 g/L at 20°C. Suitable water-soluble anti-oxidants are in particular ascorbic acid and the salts thereof, such as sodium ascorbate or zinc ascorbate. If present, the concentration of the water-soluble antioxidant is typically in the range of 0.01 to 0.5% by weight, in particular in the range of 0.02 to 0.2% by weight, based on the total weight of the composition.

Generally, the compositions of the invention have a pH, which is acceptable for oral administration. In particular, the compositions of the invention have a pH in the range of pH 5 to pH 9, as determined at 22°C and 1 bar.

The composition according to the invention is preferably essentially free of emulsifiers other than the phospholipid composition h). In this context, "essentially free" means that, based on the total mass of the components a) to d) and i), it contains no more than 50% by mass, e.g. no more than 20% by mass or no more than 10% by mass, and optimally no emulsifier other than the phospholipid composition h). In this way, undesirable side effects on the patient's digestive system (such as diarrhea) caused by the emulsifier are reduced or avoided altogether. In this context, the term "emulsifiers other than the phospholipid composition h)" refers to surface-active substances, and in particular surfactants having polyoxyethylene group with an HLB value (according to Griffin) in the range of 9-18 and especially in the range of 12-17, e. g. polysorbates (polyoxyethylene sorbitan fatty acid esters), such as polysorbate 20 (E432), polysorbate 40 (E434), polysorbate 60 (E435), polysorbate 65 (E436) and polysorbate 80 (E433) or tocopherol polyethylenglycol succinat, also referred to as Vitamin E TPGS. In particular, the amount of emulsifiers other than the phospholipid composition h) is not more than 10% by weight, based on the total weight of the phospholipid composition h) or even 0. In particular, the formulations of

The composition according to the invention is preferably free of caseinates.

The composition according to the invention is preferably essentially free of ethanol, in particular the concentration of ethanol is less than 2% by weight or even 0% by weight.

Furthermore, the composition according to the invention preferably does not contain antioxidants or preservatives selected from the classes of butylated hydroxytoluenes and butylated hydroxyanisoles, ethoxyquin, methylparaben, propylparaben, sorbic acid and sodium benzoate.

The composition according to the invention is preferably vegetarian and/or kosher and/or halal and/or allergen-free and/or gluten-free and/or free of agents that cause BSE (Bovine Spongiform Encephalopathy) and other forms of TSE (Transmissible Spongiform Encephalopathy).

Furthermore, the present invention relates to the use of the liquid compositions of the invention in the treatment of an existing or impending vitamin deficiency condition in a mammal, wherein the vitamin deficiency condition relates to one or more vitamins contained in the compositions of the invention as vitamins or in the form of corresponding provitamins.

The term "mammal" as used herein, refers to any mammalian being, including, but not limited to, human beings, but also non-human mammals, especially primates, domestic and farm animals, such as horses, pigs, cows, goats, dogs, cats etc..

The liquid compositions of the invention may be generally used for the treatment of mammals, in particular human beings, but also horses or other non-human mammals, with maldigestion and/or malabsorption causing an existing or impending vitamin deficiency condition. In particular, the composition may find application in the treatment of mammal, in particular a human being, but also horses, with age-related functional malabsorption of fat-soluble compounds, for example as a result of impaired secretion of bile or pancreatic enzymes, and in mammals suffering from diseases and conditions affecting the small intestine. Such diseases and conditions include, but are not limited to, Crohn's disease, ulcerative colitis, cystic fibrosis, cholestasis syndrome, celiac disease, indigenous sprue, liver cirrhosis, alcoholism disease, eating disorders, small bowel tumors, irritable bowel syndrome, short bowel syndrome, inflammatory bowel disease, small bowel cancer, cholestasis, malfunction of pancreas. Furthermore, the composition according to the invention can be used post-operatively after interventions on the gastrointestinal tract. According to particularly preferred embodiments, the use of the composition of the invention relate to the treatment of an existing or impending vitamin deficiency in people with cystic fibrosis, Crohn's disease, ulcerative colitis, irritable bowel syndrome or alcoholism.

The liquid compositions of the invention may also be used in the treatment of a disease or condition associated with or caused by an existing vitamin deficiency condition in a mammal, in particular human beings, but also horses or other non-human mammals, wherein the vitamin deficiency condition relates to one or more vitamins contained in the compositions of the invention as vitamins or in the form of corresponding provitamins. Disease caused by such vitamin deficiency condition include e.g. skin diseases or disorders, such as rosacea, couperose, psoriasis, neurodermatitis and vitiligo, but also skin aging, acne and keratinization disorders. In particular, the vitamins contained in the liquid compositions of the invention will stimulate cell growth and collagen formation and of antimicrobial peptides, influence keratinocyte differentiation, promote epithelialization, improve skin hydration, reduce skin redness, stabilize blood capillaries, especially the superficial capillary system. Therefore, the liquid compositions are suitable for ameliorating the above-mentioned conditions and disorders.

The liquid compositions of the invention may also be used for co-treatment of a mammal, in particular in a human being, which suffers from a disease or condition, which is treated with a therapeutic agent different from vitamin A, D, E and K, where the disease or the treatment with the therapeutic agents causes or may cause vitamin deficiency condition concerning at least one of vitamin A, D, E or K. Therapeutic agents, which cause or may cause vitamin deficiency condition concerning at least one of vitamin A, D, E or K, include e.g. chemotherapeutics used for the treatment of cancer or infectious diseases, the cholesterol lowering anion exchange resins colestyramine and colestipol, and certain antiepileptic drugs, such as phenobarbital, phenytoin, primidone or carbamazepine.

The liquid composition can be administered as such or in a suitable formulation adapted to the way of administration. Suitable formulations include, but are not limited to, formulations for oral administration, for topical administration or for parenteral administration, in particular subcutaneously or intramuscularly, e.g., as a depot. The formulation can be any formulation of a liquid and includes, besides the liquid composition of the invention, a carrier or vehicle.

The liquid composition according to the invention can be administered orally as such or in as a component of a formulation adapted for oral administration or simply diluted in water or in a non-alcoholic beverage, for example fruit juice, fruit juice diluted with mineral water, tea or milk. This makes the composition especially suitable for children and patients who are unable or unwilling to swallow ordinary tablets. However, it is also possible to use the liquid composition in dosage forms suitable for oral administrations, such as capsules. For example, the compositions can be filled in capsules of an edible material, such as soft gelatin capsules, or in hard-gelatin capsules. The liquid compositions may also be formulated in liquid vehicles suitable for oral administration. Suitable liquid vehicles include water and syrups. Therefore, a particular group of embodiments relates to a formulation for oral administration, which comprises a liquid composition as defined herein and a liquid vehicle containing the liquid composition. Another group of inventions relates to a dosage form for oral administration in the form of a capsule, in particular a soft gelatin capsule or a hard gelatin capsule containing the liquid composition as defined herein.

The liquid composition as such or a dilution thereof in a liquid vehicle, such as saline, can also be administered parenterally, in particular subcutaneously or intramuscularly, e.g., as a depot.

The liquid composition according to the invention can be administered topically, in particular if they are used for the treatment of a skin disease or disorder. For this, the liquid composition of the invention is incorporated in a vehicle suitable for topical application. Therefore, the present invention also relates to a topical formulation containing a liquid composition as defined herein and a vehicle suitable for topical application. The formulation may be a pharmaceutical formulation or a cosmetic formulation, depending on the intended use thereof. Suitable formulations include creams, ointments and lotions.

The vehicle may be any material suitable for topical formulations and include, for example, o/w emulsions, w/o emulsion, w/o/w emulsions, o/w/o emulsions, gels, fats, oils and water. Suitable ingredients for topical formulations include, for example, emollients, such as plant oils, e.g. sunflower oil, soybean oil, avocado oil, coconut oil or jojoba oil, MCT oils, and fatty acid alkyl esters, such as hexyl laurate, viscosity modifiers, such as xanthan gum or dehydro xanthan gum, polymeric gelators, such as carbomers, waxes, humectants, such as glycerin, perfume, emulsifiers, such as lecithins, mono- and diglycerides of fatty acids, alkyl polyglucosides, ethoxylated ricinolates, sorbitan fatty acid esters and ethoxylated sorbitan fatty acid esters, water and neutralizing agents, such as sodium hydroxide.

The liquid compositions of the present invention can be produced by a process, which comprises
a) mixing the components of the liquid composition and
b) homogenizing the mixture obtained in step a).

The mixture obtained in step a) is generally a so-called pre-emulsion, wherein the components a) to d), f) and optionally present i) form a first non-polar phase, and the components e) and g) form a polar phase, which optionally contains a dissolved polar component, such as a zinc salt, and where the component h) serves for the stabilization of the phases of the emulsion.

Step a) can be carried out in various ways. For example, the components a) to h) and optionally i) can be mixed in an arbitrary manner to obtain a pre-emulsion, which is then homogenized in step a).

Preferably, step a) is carried out by dissolving at least a portion of the components a) to d) and optionally i) or the total amount of the components a) to d) and optionally i) in the component f) or a portion of the component f) to obtain a solution of the respective components a) to d) and optionally i) in the component f) and then emulsify the thus obtained solution in the mixture of the components e) and g) in the presence of the component h) to obtain a pre-emulsion. The component h) may be dissolved in the solution of the respective components a) to d) and optionally i) in the component f) and then mix the thus obtain solution with the mixture of the components e) and g) to obtain the pre-emulsion. It is also possible to suspend the component h) in the mixture of the components e) and g) and mix the thus obtained suspension with the solution of the respective components a) to d) and optionally i) in the component f) to obtain the pre-emulsion. Any remaining portions of the components a) to d) and optionally i) may then be mixed with the pre-emulsion. In this case, the remaining portions of the components a) to d) and optionally i) are preferably dissolved in a portion of the component f).

According to a particular group of embodiments, the components a) to d) and optionally i) are dissolved in the component f) and the thus obtained emulsion is mixed with a suspension of the component h) in the mixture of the components e) and g).

According to another particular group of embodiments, a portion of the components a) to d) and optionally i) and the component h) are dissolved in a portion of the component f) to obtain a first solution. The first solution is then mixed with the mixture of the components e) and g) to obtain a first pre-emulsion. Then, the remaining portion of the components a) to d) and optionally i), preferably dissolved in the remaining portion of the component f), is mixed with the first pre-emulsion to obtain a second pre-emulsion.

Any zinc salt is preferably added as an aqueous solution to the pre-emulsion containing the components a) to h) and optionally i) before carrying out the homogenization step b).

Step a) is typically carried out at temperatures in the range of 10 to 80°C, in particular at temperatures in the range of 20 to 70°C. Any mixing during step a) is generally carried out by stirring or agitation of the components or by using a static mixing equipment. Step a) may be carried out batch-wise or continuously.

In step b), the pre-emulsion is then homogenized, e.g. by using a microfluidizer or a high-pressure homogenizer, to further reduce the droplet size and form the stable liquid composition of the invention. The required pressure, operating temperature, number of homogenization cycles, required for carrying out b) of the process can be found by routine based on the examples disclosed herein. Likewise, other parameters, such as type, composition and concentration the components in the pre-emulsion may influence the parameters of homogenization and the above parameters will be adjusted accordingly in a known manner.

### General remarks:

Lipoid P 75 is a commercially available phospholipid from soybean (non-GMO) containing about 70% by weight of phosphatidyl choline.

Glycerol (86%) and MCT oil (medium-chain triglycerides) had food quality. The Glycerol (86%) contained 14% of water.

For all experiments, water for injection (Ph.Eur.) with a maximum conductivity of 5.0 µS/cm was used.

Zinc-bisglycinate had 27.3% Zn content and was suitable for the use in dietary supplements.

ß-Carotin was used as a 30% suspension in sunflower oil, vitamin D₃ was used a 2.5% solution in MCT oil, vitamin K₂ was used as a 5% solution in MCT oil. Other vitamins were used in undiluted form.

High pressure homogenization was carried out with a Microfluizider 110 T (Microfluidics^{™}, USA).

pH values were determined using a calibrated portable pH meter equipped with a glass pH electrode (Portamess^{®} 911 pH, Knick GmbH, Berlin, Germany).

The mean particle size of the emulsions was determined by dynamic light scattering technique on a ZetasizerNano-ZS90 (Malvern Instruments, Malvern, UK). Emulsions were diluted with water for injection to an appropriate concentration for measurement. Measurement was performed at 25°C. The intensity-weighted mean droplet diameter is reported. For visual inspection, the emulsions were inspected in clear glass bottles.

For stability testing, the emulsions were stored in glass containers at ca. 20-25°C in the dark and in a refrigerator at +2-8°C, respectively, and analyzed at defined time points.

### Example 1: Composition of components a) to i) containing a zinc salt and sodium ascorbate

### Batch size: 600 g

| **Phase A** | | |
|---|---|---|
| Medium-chain triglycerides | 30.0000% | 180.0 g |
| Tocopherol acetate | 0.6210% | 3.73 g |
| beta-Carotin (30%) | 0.1389% | 0.83 g |
| LIPOID P 75 | 5.0000% | 30.0 g |

| **Phase B** | | |
|---|---|---|
| Sodium Ascorbate | 0.0500% | 0.30 g |
| Glycerin (86%) | 60.0251% | 360.2 g |
| Water | 2.0000% | 12.0 g |

| **Phase C** | | |
|---|---|---|
| Retinyl palmitate | 0.0061% | 37 mg |
| Vitamin K₁ | 0.0029% | 17 mg |
| Vitamin D₃ (2.5%) | 0.0033% 1) | 20 mg |

| **Phase D** | | |
|---|---|---|
| Zinc-bisglycinate | 0.1527% | 0.92 g |
| Water | 2.0000% | 12.0 g |

| | | |
|---|---|---|
| 1) % amount refers to the 2.5 solution of vitamin D₃ and corresponds to a concentration of vitamin D₃ in the formulation of 0.0000825%. | | |

Phase A: ß-Carotin was dissolved in MCT oil at 60°C. After cooling the solution to 50°C, LIPOID P 75 and tocopherol acetate were added and dissolved. To prepare phase B, sodium ascorbate was dissolved with stirring in water at 20-25°C. Glycerin was added, and the solution was heated to 50°C. Components of phase C were added to phase A with stirring to obtain a mixture AC. Phase B was added to the mixture AC and mixed for further 30 min at 50°C to obtain a mixture ABC. Zinc-bisglycinate was dissolved in water at 20-25°C, and the solution was added to the pre-emulsion ABC. The resulting pre-emulsion was then homogenized using a high-pressure homogenizer in two passes at a pressure of 900 bar. Temperature was kept at 50°C to 70°C during homogenization.

### Analytical data:

Red, transparent, slightly viscous liquid
pH value: 8.1
Average particle size (Pdl): 241 nm
Stability: 6 months at room temperature:
   Appearance unchanged
   pH value: 6.9
   Average particle size (Pdl): 271 nm
      6 month at +2-8°C:
      Appearance unchanged
      pH value: 7.1
      Average particle size (Pdl): 270 nm

### Example 2: Composition of components a) to h)

### Batch size: 300 g

| **Phase A** | | |
|---|---|---|
| Medium-chain triglycerides | 10.0000% | 30.0 g |
| Tocopherol acetate | 3.0311% | 9.09 g |
| Retinyl palmitate | 0.0373% | 0.112 g |
| Vitamin K₂ (5%) | 0.1695% ¹⁾ | 0.509 g |
| Vitamin D₃ | 0.0678% ²⁾ | 0.203 g |

| **Phase B** | | |
|---|---|---|
| LIPOID P 75 | 4.0000% | 12.0 g |

| **Phase A** | | |
|---|---|---|
| Glycerin (86%) | 82.6943% | 248.08 g |

| | | |
|---|---|---|
| 1) % amount refers to the 5% solution of vitamin K₂ and corresponds to a concentration of vitamin K₂ in the formulation of 0.008475%. 2) % amount refers to the 2.5 solution of vitamin D₃ and corresponds to a concentration of vitamin D₃ in the formulation of 0.001695%. | | |

### Phase A: The vitamins were added to MCT oil and dissolved with stirring at 20-25°C.

To prepare phase B, LIPOID P 75 was completely dispersed in glycerin with stirring at 20-30°C.

The oil phase A was added to the dispersion phase B and stirred until a homogenous mixture was obtained. The resulting pre-emulsion was then homogenized using a high-pressure homogenizer in two passes at a pressure of 900 bar. Temperature was kept at max. 50°C during homogenization.

### Analytical data:

Yellow to light brown, transparent, slightly viscous liquid
pH value: 7.0
Average particle size (Pdl): 79 nm
Stability: 4 weeks at room temperature:
   Appearance unchanged
   pH value: 6.0
   Average particle size (Pdl): 81 nm
      4 weeks at +2-8°C:
      Appearance unchanged
      pH value: 6.0
      Average particle size (Pdl): 88 nm

## Claims

1. A liquid composition containing the following components a) to h):
a) at least one of vitamin A and esters of vitamin A;
b) at least one of vitamin D and provitamin D;
c) at least one of vitamin E and esters of vitamin E;
d) at least one of vitamin K and provitamin K;
e) 40 to 95% by weight, based on the total weight of the liquid composition, of at least one water miscible polyol;
f) 1 to 40% by weight, based on the total weight of the liquid composition, of at least one edible triglyceride;
g) 3 to 30% by weight, based on the total weight of the liquid composition, water; and
h) 0.5 to 10% by weight, based on the total weight of the liquid composition, of a phospholipid composition containing phosphatidylcholine;
wherein the total amount of components a) to d) is at least 0.3% by weight, based on the total weight of the liquid composition.

2. The liquid composition of claim 1 which is translucent.

3. The liquid composition of any one of the preceding claims, wherein the total amount of components a) to d) is in the range of 0.3 to 20% by weight, based on the total weight of the composition.

4. The liquid composition of any one of the preceding claims, where the concentrations of components a) to d) are as follows:
a) 0.001 to 0.4% by weight, based on the total weight of the liquid composition, of component a),
b) 0.00005 to 0.3% by weight, based on the total weight of the liquid composition, of component b),
c) 0.1 to 19% by weight, based on the total weight of the liquid composition, of component c),
d) 0.001 to 0.4% by weight, based on the total weight of the liquid composition, of component d);
where the total concentration of components a), b), c) and d) is range of 0.3 to 20% by weight, based on the total weight of the composition.

5. The liquid composition of any one of the preceding claims further containing at least one carotenoid and/or an ester thereof as a component i), wherein the carotenoid in particular comprises ß-carotene.

6. The liquid composition of claim 5, wherein the concentration of the carotenoid in the liquid composition is in the range of 0.01 to 10% by weight, in particular in the range of 0.02 to 5% by weight, based on the total weight of the liquid composition.

7. The liquid composition of any one of the preceding claims, wherein the components a), b) c) and d) and optionally the component i) is essentially present in dissolved form.

8. The liquid composition of any one of the preceding claims further containing a zinc salt, which is suitable for nutritional or dietary purposes or pharmaceutically acceptable, wherein the zinc salt is in particular selected from zinc sulfate, zinc gluconate, zinc bisglycinate, zinc acetate, zinc hydroxide carbonate, zinc lactate, zinc ascorbate, in particular zinc L-ascorbate, zinc hydrogen ascorbate, zinc aspartate, zinc hydrogen aspartate, such as zinc DL-hydrogen aspartate or zinc L-hydrogen aspartate, zinc citrate, zinc oxide, zinc stearate, zinc orotate, zinc picolinate and the hydrates thereof.

9. The liquid composition of claim 8, wherein the concentration of the zinc salt in the liquid composition is in the range of 0.01 to 0.2% by weight, based on the total weight of the liquid composition and calculated as elemental zinc.

10. The liquid composition of any one of the preceding claims, wherein the weight ratio of the phosphatidylcholine to the triglyceride is in the range of 1:20 to 1:1.

11. The liquid composition of any one of the preceding claims, wherein the triglyceride is selected from the group consisting of oils of seed-oil plants, MCT oils, LCT oils and oils of marine organisms and combinations thereof, and where the triglyceride comprises a medium chain triglyceride as a main constituent.

12. The liquid composition of any one of the preceding claims, which has at least one of the following features a) to d), wherein
a) the weight ratio of component f) to the total weight of components a), b) c) and d) and optionally the component i) is in the range of 1:1 to 100:1, in particular in the range of 2:1 to 50:1;
b) the phospholipid composition contains phosphatidylcholine in an amount of at least 45% by weight, based on the total weight of the phospholipid composition;
c) the liquid composition is **characterized in that** a dilution of the liquid composition in deionized water at 25° C and at a concentration of 1% by weight forms droplets having a Z average particle diameter of at most 500 nm, as determined by dynamic light scattering;
d) the liquid composition additionally contains a water-soluble organic anti-oxidant, which is in particular ascorbic acid or sodium ascorbate.

13. A method for producing a liquid composition of any one of the preceding claims, which comprises
e) mixing the components of the liquid composition and
f) homogenizing the mixture obtained in step a).

14. A composition according to any one of claims 1 to 12 for use in the treatment of an existing or impending vitamin deficiency condition concerning at least one of vitamin A, D, E or K.

15. The composition of any one of claims 1 to 12 for use in a medical co-treatment of a disease with a therapeutic agent different from vitamin A, D, E and K, where the disease or the treatment with the therapeutic agents causes or may cause vitamin deficiency condition concerning at least one of vitamin A, D, E or K.

16. The use of a composition of any one of claims 1 to 12 for dietary purposes or cosmetic purposes.

## Patentansprüche

1. Flüssige Zusammensetzung, die die folgenden Komponenten a) bis h) enthält:
a) Vitamin A und/oder Ester von Vitamin A;
b) Vitamin D und/oder Provitamin D;
c) Vitamin E und/oder Ester von Vitamin E;
d) Vitamin K und/oder Provitamin K;
e) 40 bis 95 Gew.-%, bezogen auf das Gesamtgewicht der flüssigen Zusammensetzung, mindestens eines mit Wasser mischbaren Polyols;
f) 1 bis 40 Gew.-%, bezogen auf das Gesamtgewicht der flüssigen Zusammensetzung, mindestens eines essbaren Triglycerids;
g) 3 bis 30 Gew.-%, bezogen auf das Gesamtgewicht der flüssigen Zusammensetzung, Wasser und
h) 0,5 bis 10 Gew.-%, bezogen auf das Gesamtgewicht der flüssigen Zusammensetzung, einer Phosphatidylcholin enthaltenden Phospholipidzusammensetzung;
wobei die Gesamtmenge der Komponenten a) bis d) mindestens 0,3 Gew.-%, bezogen auf das Gesamtgewicht der flüssigen Zusammensetzung, beträgt.

2. Flüssige Zusammensetzung nach Anspruch 1, die transluzent ist.

3. Flüssige Zusammensetzung nach einem der vorhergehenden Ansprüche, wobei die Gesamtmenge der Komponenten a) bis d) im Bereich von 0,3 bis 20 Gew.-%, bezogen auf das Gesamtgewicht der Zusammensetzung, liegt.

4. Flüssige Zusammensetzung nach einem der vorhergehenden Ansprüche, wobei die Konzentrationen der Komponenten a) bis d) wie folgt sind:
a) 0,001 bis 0,4 Gew.-%, bezogen auf das Gesamtgewicht der flüssigen Zusammensetzung, Komponente a),
b) 0,00005 bis 0,3 Gew.-%, bezogen auf das Gesamtgewicht der flüssigen Zusammensetzung, Komponente b),
c) 0,1 bis 19 Gew.-%, bezogen auf das Gesamtgewicht der flüssigen Zusammensetzung, Komponente c),
d) 0,001 bis 0,4 Gew.-%, bezogen auf das Gesamtgewicht der flüssigen Zusammensetzung, Komponente d);
wobei die Gesamtkonzentration der Komponenten a), b), c) und d) im Bereich von 0,3 bis 20 Gew.-%, bezogen auf das Gesamtgewicht der Zusammensetzung, liegt.

5. Flüssige Zusammensetzung nach einem der vorhergehenden Ansprüche, die ferner mindestens ein Carotinoid und/oder einen Ester davon als Komponente i) enthält, wobei es sich bei dem Carotinoid insbesondere um β-Carotin handelt.

6. Flüssige Zusammensetzung nach Anspruch 5, wobei die Konzentration des Carotinoids in der flüssigen Zusammensetzung im Bereich von 0,01 bis 10 Gew.-%, insbesondere im Bereich von 0,02 bis 5 Gew.-%, bezogen auf das Gesamtgewicht der flüssigen Zusammensetzung, liegt.

7. Flüssige Zusammensetzung nach einem der vorhergehenden Ansprüche, wobei die Komponenten a), b) c) und d) und gegebenenfalls die Komponente i) im Wesentlichen in gelöster Form vorliegen.

8. Flüssige Zusammensetzung nach einem der vorhergehenden Ansprüche, die ferner ein Zinksalz enthält, das für Ernährungszwecke oder diätetische Zwecke geeignet oder pharmazeutisch unbedenklich ist, wobei das Zinksalz insbesondere aus Zinksulfat, Zinkgluconat, Zinkbisglycinat, Zinkacetat, Zinkhydroxidcarbonat, Zinklactat, Zinkascorbat, insbesondere Zink-L-Ascorbat, Zinkhydrogenascorbat, Zinkaspartat, Zinkhydrogenaspartat, wie Zink-DL-hydrogenaspartat oder Zink-L-hydrogenaspartat, Zinkcitrat, Zinkoxid, Zinkstearat, Zinkorotat, Zinkpicolinat und den Hydraten davon ausgewählt ist.

9. Flüssige Zusammensetzung nach Anspruch 8, wobei die Konzentration des Zinksalzes in der flüssigen Zusammensetzung im Bereich von 0,01 bis 0,2 Gew.-%, bezogen auf das Gesamtgewicht der flüssigen Zusammensetzung und berechnet als elementares Zink, liegt.

10. Flüssige Zusammensetzung nach einem der vorhergehenden Ansprüche, wobei das Gewichtsverhältnis von Phosphatidylcholin zu Triglycerid im Bereich von 1:20 bis 1:1 liegt.

11. Flüssige Zusammensetzung nach einem der vorhergehenden Ansprüche, wobei das Triglycerid aus der Gruppe bestehend aus Ölen von Samenölpflanzen, MCT-Ölen, LCT-Ölen und Ölen von Meeresorganismen und Kombinationen davon ausgewählt ist und wobei das Triglycerid ein mittelkettiges Triglycerid als Hauptbestandteil umfasst.

12. Flüssige Zusammensetzung nach einem der vorhergehenden Ansprüche, die mindestens eines der folgenden Merkmale a) bis d) aufweist, wobei
a) das Gewichtsverhältnis von Komponente f) zum Gesamtgewicht der Komponenten a), b) c) und d) und gegebenenfalls der Komponente i) im Bereich von 1:1 bis 100:1, insbesondere im Bereich von 2:1 bis 50:1, liegt;
b) die Phospholipidzusammensetzung Phosphatidylcholin in einer Menge von mindestens 45 Gew.-%, bezogen auf das Gesamtgewicht der Phospholipidzusammensetzung, enthält;
c) die flüssige Zusammensetzung **dadurch gekennzeichnet ist, dass** eine Verdünnung der flüssigen Zusammensetzung in vollentsalztem Wasser bei 25 °C und einer Konzentration von 1 Gew.-% Tröpfchen mit einem Z-mittleren Teilchendurchmesser von höchstens 500 nm bildet, wie durch dynamische Lichtstreuung bestimmt;
d) die flüssige Zusammensetzung zusätzlich ein wasserlösliches organisches Antioxidans enthält, bei dem es sich insbesondere um Ascorbinsäure oder Natriumascorbat handelt.

13. Verfahren zur Herstellung einer flüssigen Zusammensetzung nach einem der vorhergehenden Ansprüche, das Folgendes umfasst
e) Mischen der Komponenten der flüssigen Zusammensetzung und
f) Homogenisieren der in Schritt a) erhaltenen Mischung.

14. Zusammensetzung nach einem der Ansprüche 1 bis 12 zur Verwendung bei der Behandlung eines bestehenden oder bevorstehenden Vitaminmangelzustands bezüglich Vitamin A, D, E und/oder K.

15. Zusammensetzung nach einem der Ansprüche 1 bis 12 zur Verwendung bei einer medizinischen Begleitbehandlung einer Krankheit mit einem von Vitamin A, D, E und K verschiedenen therapeutischen Mittel, wobei die Krankheit oder die Behandlung mit den therapeutischen Mitteln einen Vitaminmangelzustand bezüglich Vitamin A, D, E und/oder K verursacht oder verursachen kann.

16. Verwendung einer Zusammensetzung nach einem der Ansprüche 1 bis 12 für diätetische Zwecke oder kosmetische Zwecke.

## Revendications

1. Composition liquide contenant les composants a) à h) suivants :
a) au moins l'un parmi la vitamine A et des esters de vitamine A ;
b) au moins l'une parmi la vitamine D et la provitamine D ;
c) au moins l'un parmi la vitamine E et des esters de vitamine E ;
d) au moins l'une parmi la vitamine K et la provitamine K ;
e) 40 à 95 % en poids, sur la base du poids total de la composition liquide, d'au moins un polyol miscible à l'eau ;
f) 1 à 40 % en poids, sur la base du poids total de la composition liquide, d'au moins un triglycéride comestible ;
g) 3 à 30 % en poids, sur la base du poids total de la composition liquide, d'eau ; et
h) 0,5 à 10 % en poids, sur la base du poids total de la composition liquide, d'une composition de phospholipides contenant de la phosphatidylcholine ;
dans laquelle la quantité totale des composants a) à d) est d'au moins 0,3 % en poids, sur la base du poids total de la composition liquide.

2. Composition liquide selon la revendication 1, qui est translucide.

3. Composition liquide selon l'une quelconque des revendications précédentes, dans laquelle la quantité totale des composants a) à d) est dans la plage de 0,3 à 20 % en poids, sur la base du poids total de la composition.

4. Composition liquide selon l'une quelconque des revendications précédentes, où les concentrations des composants a) à d) sont comme suit :
a) 0,001 à 0,4 % en poids, sur la base du poids total de la composition liquide, du composant a),
b) 0,00005 à 0,3 % en poids, sur la base du poids total de la composition liquide, du composant b),
c) 0,1 à 19 % en poids, sur la base du poids total de la composition liquide, du composant c),
d) 0,001 à 0,4 % en poids, sur la base du poids total de la composition liquide, du composant d) ;
où la concentration totale des composants a), b), c) et d) est dans la plage de 0,3 à 20 % en poids, sur la base du poids total de la composition.

5. Composition liquide selon l'une quelconque des revendications précédentes, contenant en outre au moins un caroténoïde et/ou un ester de celui-ci en tant que composant i), dans laquelle le caroténoïde comprend en particulier du β-carotène.

6. Composition liquide selon la revendication 5, dans laquelle la concentration du caroténoïde dans la composition liquide est dans la plage de 0,01 à 10 % en poids, en particulier dans la plage de 0,02 à 5 % en poids, sur la base du poids total de la composition liquide.

7. Composition liquide selon l'une quelconque des revendications précédentes, dans laquelle les composants a), b) c) et d) et éventuellement le composant i) sont essentiellement présents sous forme dissoute.

8. Composition liquide selon l'une quelconque des revendications précédentes, contenant en outre un sel de zinc, qui convient à des fins nutritionnelles ou diététiques ou est pharmaceutiquement acceptable, dans laquelle le sel de zinc est choisi en particulier parmi le sulfate de zinc, le gluconate de zinc, le bisglycinate de zinc, l'acétate de zinc, l'hydroxyde carbonate de zinc, le lactate de zinc, l'ascorbate de zinc, en particulier le L-ascorbate de zinc, l'hydrogéno-ascorbate de zinc, l'aspartate de zinc, l'hydrogéno-aspartate de zinc, tel que le DL-hydrogéno-aspartate de zinc ou le L-hydrogéno-aspartate de zinc, le citrate de zinc, l'oxyde de zinc, le stéarate de zinc, l'orotate de zinc, le picolinate de zinc et leurs hydrates.

9. Composition liquide selon la revendication 8, dans laquelle la concentration du sel de zinc dans la composition liquide est dans la plage de 0,01 à 0,2 % en poids, sur la base du poids total de la composition liquide et calculée comme zinc élémentaire.

10. Composition liquide selon l'une quelconque des revendications précédentes, dans laquelle le rapport pondéral de la phosphatidylcholine sur le triglycéride est dans la plage de 1:20 à 1:1.

11. Composition liquide selon l'une quelconque des revendications précédentes, dans laquelle le triglycéride est choisi dans le groupe constitué des huiles de plantes grainées-oléagineuses, des huiles MCT, des huiles LCT et des huiles d'organismes marins et leurs combinaisons, et dans laquelle le triglycéride comprend un triglycéride à chaîne moyenne en tant que constituant principal.

12. Composition liquide selon l'une quelconque des revendications précédentes, qui présente au moins l'une des caractéristiques suivantes a) à d), dans laquelle
a) le rapport pondéral du composant f) sur le poids total des composants a), b), c) et d) et éventuellement le composant i) est dans la plage de 1:1 à 100:1, en particulier dans la plage de 2:1 à 50:1 ;
b) la composition de phospholipides contient de la phosphatidylcholine en une quantité d'au moins 45 % en poids sur la base du poids total de la composition de phospholipides ;
c) la composition liquide est **caractérisée en ce qu'**une dilution de la composition liquide dans de l'eau désionisée à 25 °C et à une concentration de 1 % en poids forme des gouttelettes ayant un diamètre de particule moyen Z d'au plus 500 nm, tel que déterminé par diffusion dynamique de lumière ;
d) la composition liquide contient en outre un antioxydant organique hydrosoluble, qui est en particulier l'acide ascorbique ou l'ascorbate de sodium.

13. Procédé de production d'une composition liquide selon l'une quelconque des revendications précédentes, qui comprend
e) le mélange des composants de la composition liquide et
f) l'homogénéisation du mélange obtenu à l'étape a).

14. Composition selon l'une quelconque des revendications 1 à 12, destinée à être utilisée dans le traitement d'un état de carence vitaminique existant ou imminent concernant au moins l'une des vitamines A, D, E ou K.

15. Composition selon l'une quelconque des revendications 1 à 12, destinée à être utilisée dans un co-traitement médical d'une maladie avec un agent thérapeutique différent de la vitamine A, D, E et K, où la maladie ou le traitement avec les agents thérapeutiques provoque ou peut provoquer un état de carence vitaminique concernant au moins l'une des vitamines A, D, E ou K.

16. Utilisation d'une composition selon l'une quelconque des revendications 1 à 12 à des fins diététiques ou des fins cosmétiques.
